# EUROPÄISCHE PATENTANMELDUNG

(11) **EP 1 116 485 A2**
(43) Veröffentlichungstag der Anmeldung: **18.07.2001**
(21) Anmeldenummer: 00101924.9
(22) Anmeldetag: 01.02.2000
(51) Int. Cl.: A61K 9/16

(54) **Instant-Granulat und Verfahren zu seiner Herstellung**

(30) Priorität: 10.01.2000 CH 33002000
(71) Anmelder: Gergely, Gerhard, Dr., A-1053 Wien (AT)
(72) Erfinder: Gergely, Gerhard, Dr., 1053 Wien (AT); Gergely, Irmgard, 1053 Wien (AT)
(74) Vertreter: Büchel, Kurt F., Dr.

(57) **Zusammenfassung**

Das Instantgranulat enthält Körner wenigstens eines löslichen Trägermaterials, deren Oberfläche von wenigstens einer, wenigstens einen Wirkstoff enthaltenden Schicht abgedeckt bzw. überzogen ist. Als Trägermaterial kann zumindest wenigstens ein Zuckeralkohol, insbesondere Mannit oder Sorbit, und wenigstens ein granuliertes, lösliches Hydrokolloid, insbesondere Maltodextrin dienen. Vorzugsweise sind etwa 50 bis etwa 80 Gew.% des Trägermaterials mit einer Schicht abgedeckt bzw. überzogen, die nur einen Teil des Wirkstoffes enthält, während das restliche Trägermaterial mit dem restlichen Wirkstoff beschichtet und/oder gemeinsam mit dem ersten beschichteten Trägermaterial granuliert vorliegt. Die Gesamtmenge Wirkstoff kann in diesem Fall 50 bis 120, vorzugsweise 60 bis 100 Gew.teile Wirkstoff - insbesondere (auch mehrere verschiedene) - Aminosäuren, auf 100 Gew.teile Trägermaterial betragen, wobei der Wirkstoff vorzugsweise eine Korngrössenverteilung aufweist, bei der 10 bis 50 Gew.% zwischen 0,1 und 0,2 mm, sowie 20 bis 70 Gew.% unter 0,1 mm liegen.

## Beschreibung

Die Erfindung betrifft eine pharmazeutische Zubereitung in Form eines Instantgranulates nach dem Oberbegriff des Anspruchs 1. Eine solche Zubereitung ist beispielsweise in der EP-B1-232.277 beschrieben. Dort ist bereits die Rede davon, daß als Wirkstoff eine Aminosäure (z.B. Tryptophan in Beispiel 1) oder mehrere Wirkstoffe (z.B. Vitamine und Mineralsalze in Beispiel 2, allerdings nicht beansprucht, weil auf ein Trägermaterial aus Säure aufgebracht, die für Nieren-Patienten ein Problem bedeutet) erfindungsgemäß verarbeitet werden können, wobei auch angegeben wird, daß zur Verankerung größerer Wirkstoffmengen mehrere Schichten mit einer Bindemittellösung auf das körnige Trägermaterial aus löslichem Kohlehydrat aufgetragen werden können.

Dieser letzte Vorschlag stößt in der Praxis, speziell bei Aminosäuren, an Grenzen, wenn nicht besondere Maßnahmen getroffen werden. Insbesondere hat sich herausgestellt, daß für Wirkstoffmengen, die über die in den dort genannten Beispielen vorkommenden Mengen hinausgehen und die sehr voluminös sind, die Wirkstoffe auf den Kohlehydratträgern durch die Bindemittelschicht allein nicht ausreichend verankert werden können und zuviel Wirkstoff frei bliebe, wodurch weder die gewünschte Suspensionseigenschaft noch die Fließfähigkeit des Produktes zufriedenstellend erreicht werden kann.

Bei Wirkstoffmengen von 60 bis 100 Gew.Teilen - auf 100 Gew.Teile Träger bezogen - kommt es bei mehrfacher Aufbringung des Bindemittels zu unerwünschten Agglomerationen des Wirkstoffes, so daß beim Einbringen in Wasser nicht mehr die gewünschte Suspension erzielbar ist und die Teilchen zu Boden sinken.

Die Erfindung hat sich daher die Aufgabe gestellt, die in der genannten EP beschriebene Zubereitung und deren Herstellung dahingehend zu verbessern, dass Instantgranulate und ein Verfahren zu deren Herstellung geschaffen werden, mit dem es möglich ist, auch sehr große Mengen auch verschiedener - insbesondere unlöslicher oder schwerlöslicher - Wirkstoffe, insbesondere mehrere Aminosäuren, Analgetika, Antioxidantien, wie z.B. Paracetamol, Beta-Carotin etc., problemlos unterzubringen. Die pharmazeutische Zubereitung soll nach dem Einbringen in Wasser durch Rühren innerhalb angemessener Zeit suspendiert werden, gut schmecken und sich des weiteren eine angemessene Zeit in Suspension halten.

Diese Forderung gilt deswegen besonders für Aminosäuren, da diese in großen Mengen pro Dosis verabreicht werden müssen, sowohl bei der Anwendung als Nahrungsergänzungsmittel, als auch insbesondere bei Nierenpatienten, die mit einer niedrigen Proteindiät zu leben haben und eine hohe Zufuhr von Aminosäuren benötigen. Durch die Zufuhr von essentiellen Aminosäuren kann das Defizit gut ausgeglichen werden, wodurch negative Begleiterscheinungen verhindert und die Notwendigkeit einer Dialyse unter Umständen hinausgeschoben werden kann.

Nun gibt es eine Reihe von aminosäurehältigen Produkten mit essentiellen Aminosäuren in Tabletten- oder Kapselform auf dem Markt, doch müssen davon z.B. von Nierenpatienten 3x täglich 5 Stück eingenommen werden, was den Patienten oder Konsumenten erhebliche Probleme bereitet. Ziel der Erfindung war es daher, eine Instant-Zubereitung mit einer hohen Wirkstoffdosis und geringen Mengen an Hilfsstoffen zu entwickeln, die sich in einer geringen Wassermenge von 70 bis 100 ml gut suspendieren läßt und vom Patienten geschmacklich gut akzeptiert wird. Damit kann die Einnahme für den Patienten wesentlich erleichtert werden.

Dies gelingt erfindungsgemäß durch die Verwirklichung der im Kennzeichen des Anspruchs 1 genannten Merkmale, und vorzugsweise mittels der im Verfahrensanspruch genannten, kennzeichnenden Merkmale. Vorteilhafte Ausführungsformen der Erfindung sind in den abhängigen Ansprüchen beschrieben.

Der Weg zu dieser Erfindung ging von der Überlegung aus, daß in Fällen, in denen geringe Hilfsstoffmengen erwünscht sind, sei es aus diätetischer oder medizinischer Indikation oder auch aus ökonomischen Gründen, eine Vorgangsweise zu suchen ist, die es ermöglicht, voluminöse unlösliche oder schwer lösliche Wirkstoffe in einem Gewichtsverhältnis von 60 bis 100 Gew.Teilen - bezogen auf 100 Gew.Teile Trägerstoff - herzustellen.

Dies gelingt, indem man die Trägerstoffe in zwei oder mehreren Stufen einbringt. Im ersten Schritt wird ein Teil der Trägerstoffe vorgelegt; man benetzt dann ihre Oberfläche durch Zugabe von Wasser, von einer Wasser/Alkoholmischung oder einer Bindemittellösung. Dann werden die Wirkstoffe zugefügt und wenigstens teilweise an der Kornoberfläche verankert. Die restlichen, noch nicht verankerten Wirkstoffpartikel werden in einem zweiten Schritt durch Einbringen eines weiteren löslichen Trägerstoffes und durch Anlösen seiner Oberfläche mittels der in der Masse vorhandenen Restfeuchte - oder erforderlichenfalls unter - insbesondere vorheriger - Zugabe von weiterem Wasser, einer Wasser/Alkoholmischung oder einer Bindemittellösung - daran verankert oder mit dem löslichen Trägerstoff granuliert. Dieser Schritt kann bei Bedarf wiederholt werden.

In der Praxis hat sich gezeigt, dass mit einer Flüssigkeitsmenge von etwa 3 bis etwa 7 Gew.teilen (auf 100 Gew.teile Trägermaterial und Wirkstoff bezogen) die besten Ergebnisse erzielt werden. Die untere Grenze gilt beim Einsatz von reinem Wasser und weitgehend unlöslichen bzw. sehr schwer löslichen Wirkstoffen, ein mittlerer Wert ist bei Alkohol-Wasser-Gemischen und noch wenig löslichen Wirkstoffen anzunehmen; die obere Grenze gilt beim Einsatz einer Bindemittellösung.

Dabei hat es sich weiter als zweckmässig erwiesen, wenn zunächst nur 10 bis 25, insbesondere 15 bis 20 Gew.% der vorgesehenen Gesamtmenge an Flüssigkeit zur Benetzung des ersten Trägermaterials herangezogen werden. Dieses soll nämlich im Wesentlichen befeuchtet, aber nur minimal angelöst werden, da die Masse ansonsten die Tendenz hat zusammenzukleben, wodurch eine gleichmässige Verteilung, nämlich das gleichmässige Aufbringen des Wirkstoffes auf die einzelnen Trägerkristalle erschwert wird.

Die Restmenge an Flüssigkeit wird dann zur Benetzung des restlichen Wirkstoffes und des zweiten Trägermaterials verwendet, das vorzugsweise leichter löslich ist als das erste. Dabei hat es sich in vielen Fällen aus demselben Grund, nämlich der Vermeidung des Zusammenklebens des Trägermaterials, als zweckmässig erwiesen, die Restmenge an Flüssigkeit auf dem ersten, schon mit Wirkstoffpartikeln abgedeckten Trägermaterial, sowie auf den freien Wirkstoffpartikeln v o r der Zugabe des zweiten Trägermaterials zu verteilen. Man erzielt dann je nach Art der Bestandteile und der Vorgehensweise analog mit Wirkstoff beschichtete Körner des zweiten Trägermaterials und/oder mit Wirkstoff und Trägermaterial granulierte Teilchen.

Dabei kann für eine Formulierung, bei der aus 70 bis 80 Gew.Teilen voluminöser, nichtlöslicher oder schwer löslicher Wirkstoffe auf 100 Gew.Teilen Trägerstoff ein frei fließendes, suspendierfähiges Granulat erzielt werden soll, folgendermaßen vorgegangen werden:

Im ersten Schritt werden 60 bis 80 % des zur Verfügung stehenden Trägermaterials mit z.B. einer Bindemittellösung benetzt. Die Bindemittellösung kann eine wässrige oder alkoholische oder alkoholisch-wässrige Lösung von wenigstens einer der folgenden Substanzen sein: Zucker, Zuckeralkohol, Maltodextrin, Polyvinylpyrrolidon oder andere Hydrokolloide.

Dann werden die Wirkstoffe zugefügt und wenigstens teilweise an der Oberfläche der Trägerpartikel verankert. Sodann werden die restlichen Wirkstoffpartikel mittels einer weiteren Bindemittellösung untereinander verankert und benetzt.

Es erfolgt darauf in einem zweiten Schritt die Zugabe des restlichen, zweiten Trägermaterials, wobei sich dafür besonders sprühgetrocknetes Sorbit oder Maltodextrin - auf Grund ihres Schüttgewichtes, der zerklüfteten, voluminösen Struktur und des guten Anlösungsvermögens an der Oberfläche - eignen. Durch die in der Masse bereits vorhandene - oder gegebenenfalls wie erwähnt zusätzlich zugeführte - Feuchtigkeit löst sich nun auch die Oberfläche der weiteren Trägerstoffpartikel an und bindet die noch frei liegenden Wirkstoffe einerseits an der Oberfläche; anderseits dienen sie als Kern für die Granulierung des Wirkstoffes mit dem Vorteil, daß beim Einbringen in Wasser sich der Kern löst, die Wirkstoffpartikel frei werden und so eine gute Suspension ermöglicht wird. Das Einbringen der Trägermaterialien kann auch in mehreren Schritten erfolgen.

Als Zuckeralkohole können Sorbit, Mannit und Xylit Verwendung finden. Die Trägermaterialien sollen in einer Korngröße von 0,4 bis 0,1 mm vorliegen, wobei eine Hauptsiebfraktion zwischen 0,4 und 0,2 mm vorteilhaft ist. Sie können sowohl in kristalliner Form als auch als sprühgetrockneter oder granulierter Rohstoff vorliegen. Bei den Wirkstoffen empfiehlt sich eine Korngröße, bei der 10 bis 50 Gew.% zwischen 0,1 und 0,2 mm, sowie 20 bis 70 Gew.% unter 0,1 mm liegen. Um eine Verbesserung der Suspension zu erzielen, können noch Suspendierhilfsmittel oder Benetzungsmittel eingesetzt werden. Dafür eignen sich sowohl anionische Tenside wie Natriumlaurylsulfat oder Dioctylsulfosuccinat, aber auch Zuckerester, Phospholipide, Polysorbate oder hydrogenierte Rhizinusöle.

Der Zubereitung können zur Geschmacksverbesserung künstliche Süßstoffe sowie Aromen und Säuren oder deren saure Salze in der erlaubten oder für die Patientengruppe zulässigen Menge, bzw. auch Vitamine zugefügt werden.

Eine besondere Formulierung stellt eine Aminosäure-Instant-Zubereitung für Nierenpatienten dar. Es muß dabei aus medizinischen Gründen speziell auf die Zusammensetzung der Formulierung geachtet werden. Es soll z.B. eine Dosis, die dreimal am Tag verabreicht wird, möglichst wenig Zucker, sowie Mannit oder Sorbit in einer Menge von maximal 1,5 g enthalten, weiters eine möglichst geringe Menge an organischen Säuren, wie Zitronensäure oder Weinsäure bzw. deren sauren Salzen. Unerwünscht sind weiters phosphathältige Hilfsstoffe. Die essentiellen Aminosäuren, das sind L-Valin, L-lsoleucin, L-Tyrosin, L-Threonin, L-Methionin und L-Lysin, müssen in entsprechender Menge pro Dosis enthalten sein, wobei der Zusatz einiger weiterer Aminosäuren zweckmässig ist, wie insbesondere L-Leucin, L-Histidin, L-Tryptophan und L-Phenylalanin. Wird der Bedarf eines Tages auf drei Dosen aufgeteilt, haben sich z.B. 450 mg L-Leucin, 675 mg L-Valin, 300 mg L-lsoleucin, 325 mg L-Histidin, 375 mg L-Tyrosin, 125 mg L-Tryptophan, 325 mg L-Threonin, 450 mg L-Methionin, 350 mg L-Phenylalanin und 480 mg L-Lysinacetat pro Dosis als zweckmässig erwiesen. Ein Instantgranulat mit dieser Dosis ist ohne weiteres in 70-80 ml Wasser suspendierbar und ist - zusammen mit Süss- und Aromastoffen - angenehm zu trinken. Will man die dreifache Menge in einer einzigen Tagesdosis verabreichen, sind dazu 200-250 ml Wasser erforderlich.

Diese technisch und geschmacklich schwierigen Forderungen konnten mit den erfindungsgemäßen Massnahmen und mit einem Verfahren zur Herstellung der erfindungsgemäßen Instant-Zubereitung gelöst werden.

Das Verfahren zur Herstellung der erfindungsgemässen Instantgranulate geht von der Überlegung aus, dass - einerseits wegen der bevorzugten, grossen Mengen an Wirkstoff, andererseits auch wegen des allfälligen Einsatzes von nämlich besonders leicht und schnell löslichen Trägermaterialien wie z.B. Sorbit oder Maltodextrin - zweistufig vorgegangen werden muss. Unter bestimmten Umständen kann es dann sogar zweckmässig sein, jedenfalls die zweite Bindemittellösung auf Basis Ethanol oder bestenfalls einer Ethanol-Wasser-Mischung einzusetzen, wenn weitere Wirkstoffmengen aufgebracht werden sollen.

### Beispiel 1:

Man bringt 930 g Mannit Feingries, 793 g Feinkristallzucker und 18 g Natriumchlorid, gegebenenfalls mit künstlichen Süßstoffen, in einen Mischkessel, vorzugsweise einen Vakuumkessel, und heizt unter Mischen auf 60° C auf. Anschließend legt man Vakuum an und saugt 40 ml (=46.1 g) einer Lösung aus 27 ml Wasser, 18 g Zucker und 1,1 g Zitronensäure ein, verteilt diese und fügt dann 1965 g einer Mischung der gewünschten Aminosäuren hinzu.

Anschließend saugt man - vorzugsweise unter Vakuum - 200 ml (= 227.4 g) derselben Lösung ein, die 133 ml Wasser, 89 g Sucrose und 5,4 g Zitronensäure enthält, verteilt die Lösung und mischt anschließend 900 g Maltodextrin zu. Durch die nun in der Masse vorhandene Feuchtigkeit löst sich das Maltodextrin an und bindet die freiliegenden Wirkstoffpartikel. Man saugt dann 180 ml einer Lösung ein, die aus 150 ml Ethanol und 21 g Epikuron® (Phospholipide) besteht und in der Masse verteilt wird. Zuletzt bringt man noch 245 g eines Zitronensäure/Weinsäure-Gemisches, sowie gegebenenfalls 288 g einer weiteren, z.B. grobkörnigen, löslichen Aminosäure wie etwa L-Lysinacetat, und 48 g eines Zitronenaromas ein. Nach der Endtrocknung wird das Granulat auf 1,2 mm gesiebt.

Durch diese Vorgangsweise erhält man ein Instantgranulat, das pro Dosis von 8,94 g eine Menge von 3,75 g Aminosäuren enthält, in 75 ml Wasser gut suspendierbar ist und gut schmeckt.

### Beispiel 2: Zuckerfreie Formulierung

Man bringt 1750 g Mannit Feingries und 18 g Natriumchlorid, gegebenenfalls mit künstlichen Süßstoffen, in einen Mischkessel, vorzugsweise einen Vakuumkessel, und heizt unter Mischen auf 60° C auf. Anschließend legt man Vakuum an und saugt 40 ml einer Lösung aus 27 ml Wasser, 18 g Sorbit und 1,1 g Zitronensäure ein, verteilt diese und fügt dann 1965 g einer Mischung der gewünschten Aminosäuren hinzu.

Anschließend saugt man - vorzugsweise unter Vakuum - 220 ml derselben Lösung (enthaltend 145 ml Wasser, 98 g Sorbit und 5,4 g Zitronensäure) ein, verteilt die Lösung und mischt anschließend 800 g sprühgetrocknetes Sorbit zu. Durch die in der Masse vorhandene Feuchtigkeit löst sich das Sorbit an und bindet die freiliegenden Wirkstoffpartikel. Man saugt dann 160 ml einer Lösung ein, die aus 130 ml Ethanol und 21 g Epikuron® (Phospholipide) besteht und in der Masse verteilt wird. Zuletzt bringt man noch 245 g eines Zitronensäure/Weinsäure-Gemisches, sowie gegebenenfalls 288 g einer weiteren, z.B. grobkörnigen löslichen Aminosäure wie etwa Lysinacetat, und 48 g eines Zitronenaromas ein. Nach der Endtrocknung wird das Granulat auf 1,2 mm gesiebt.

Durch diese Vorgangsweise erhält man ein zuckerfreies Instantgranulat, das pro Dosis von 8,85 g eine Menge von 3,75 g Aminosäuren enthält, in 75 ml Wasser gut suspendierbar ist und gut schmeckt.

### Beispiel 3: Antioxidans

500 Gew.Teile Saccharose Feingries und 866 Gew.Teile sprühgetrocknetes Mannit, sowie 12 Gew.Teile Saccharin werden unter Mischen auf 60° C erwärmt. Diese Trägermaterialien werden mit 30 ml Wasser benetzt. Anschließend bringt man die Wirkstoffe, bestehend aus 1000 Gew.Teilen Vitamin E Trockenpulver 50 %ig und 120 Gew.Teile Beta-Carotin 10 %ig ein. Darauf verteilt man eine Lösung von 50 Gew.Teilen Sorbit in 100 Gew.Teilen Ethanol und bringt sodann 500 Gew.Teile Instantzucker ein. Das Produkt wird getrocknet und zu einem Granulat gemischt, das aus 100 Gew.Teilen Zitronensäure, 150 Gew.Teilen Vitamin C , 25 Gew.Teilen Natriumcarbonat und einer gewünschten Menge Aroma besteht.

Das Produkt mit 65 Gew.Teilen Wirkstoff auf 100 Gew.Teilen Träger ergibt ein in Wasser gut suspendierbares und geschmacklich angenehmes Produkt.

## Patentansprüche

1. Pharmazeutische Zubereitung in Form eines Instantgranulates, bei dem die Oberfläche der Körner wenigstens zweier löslicher Trägermaterialien von wenigstens einer Schicht abgedeckt bzw. überzogen ist, die wenigstens einen - vorzugsweise unlöslichen oder schwerlöslichen - Wirkstoff enthält, gekennzeichnet durch wenigstens eine der folgenden Massnahmen:
a) als Trägermaterial liegt wenigstens ein Zuckeralkohol, insbesondere Mannit oder Sorbit, und wenigstens ein lösliches Hydrokolloid, insbesondere Maltodextrin, vor, und auf 100 Gew.teile Trägermaterial liegen insgesamt 50 bis 120, vorzugsweise 60 bis 100 Gew.teile Wirkstoff vor;
b) etwa 50 bis etwa 80 Gew.% eines ersten Trägermaterials sind mit einer Schicht abgedeckt bzw. überzogen, die nur einen Teil des Wirkstoffes enthält, während das restliche, zweite Trägermaterial leichter löslich ist als das erste und teilweise mit dem restlichen Wirkstoff abgedeckt und granuliert vorliegt.

2. Zubereitung nach Anspruch 1, dadurch gekennzeichnet, dass der Wirkstoff eine Korngrössenverteilung aufweist, bei der 10 bis 50 Gew.% zwischen 0,1 und 0,2 mm, sowie 20 bis 70 Gew.% unter 0,1 mm liegen.

3. Zubereitung nach Anspruch 1 oder 2, dadurch gekennzeichnet, dass als Wirkstoff eine Mischung essentieller Aminosäuren, d.i. L-Valin, L-lsoleucin, L-Tyrosin, L-Threonin, L-Methionin und L-Lysin vorliegt, gegebenenfalls unter Zusatz von wenigstens einer der folgenden Aminosäuren: L-Leucin, L-Histidin, L-Tryptophan und L-Phenylalanin.

4. Zubereitung nach Anspruch 3, dadurch gekennzeichnet, dass sie in Sachets in einer solchen Menge vorliegt, dass in 1 bis 3 Dosen pro Tag
1800 - 2700, vorzugsweise etwa 2025 mg L-Valin;
750 - 1200, vorzugsweise etwa 900 mg L-lsoleucin;
1000 - 1500, vorzugsweise etwa 1125 mg L-Tyrosin;
800 - 1300, vorzugsweise etwa 975 mg L-Threonin;
1200 - 1800, vorzugsweise etwa 1350 mg L-Methionin;
800 - 1500, vorzugsweise etwa 1300 mg L-Lysinacetat,
sowie gegebenenfalls wenigstens eine der folgenden Aminosäuren:
1200 - 1800, vorzugsweise etwa 1350 mg L-Leucin;
600 - 1200, vorzugsweise etwa 900 mg L-Histidin;
300 - 500, vorzugsweise etwa 375 mg L-Tryptophan, und
900 - 1400, vorzugsweise etwa 1050 mg L-Phenylalanin
enthalten sind.

5. Verfahren zur Herstellung einer pharmazeutischen Zubereitung nach einem der vorhergehenden Ansprüche, dadurch gekennzeichnet, dass ein Zuckeralkohol, vorzugsweise Mannit oder Sorbit, gegebenenfalls in Mischung mit einem Zucker, vorzugsweise Saccharose, mit wenigstens einem Teil der vorgesehenen Gesamtmenge von Wasser, eines Alkohol-Wasser-Gemisches oder der wässrigen Lösung eines Bindemittels, die gegebenenfalls eine essbare organische Säure enthält, benetzt wird, worauf die körnigen oder pulvrigen Wirkstoffe eingebracht und mit den Trägerkörnern vermischt werden, worauf der allfällige restliche Teil des Wassers bzw. des Alkohol-Wasser-Gemisches bzw. der Lösung und danach ein weiteres Trägermaterial - vorzugsweise Maltodextrin - eingebracht und mit der Masse vermischt werden, während anschliessend getrocknet und schliesslich auf die gewünschte Korngrösse vermahlen und/oder gesiebt wird.

6. Verfahren zur Herstellung einer pharmazeutischen Zubereitung nach einem der Ansprüche 1 bis 4, gekennzeichnet durch die folgenden - vorzugsweise in einem Vakuum-Mischer durchgeführten - Verfahrensschritte:
a) 60 bis 80 Gew.% des Trägermaterials werden mit wenigstens einem Teil - vorzugsweise mit 10 bis 25, insbesondere 15 bis 20 Gew.% - der vorgesehenen Gesamtmenge von Wasser, eines Alkohol-Wasser-Gemisches oder einer Bindemittellösung benetzt;
b) einer oder mehrere pulvrige Wirkstoffe - sowie gegebenenfalls anschließend der restliche Teil des Wassers, des Alkohol-Wasser-Gemisches bzw. der Lösung - werden aufgebracht;
c) der Rest des Trägermaterials wird eingebracht und mit der Masse zur Abbindung des noch nicht gebundenen Wirkstoffes vermischt und wenigstens teilweise getrocknet.

7. Verfahren nach Anspruch 6, dadurch gekennzeichnet, dass die Mischung aller Bestandteile zunächst nur teilweise getrocknet und ihr danach wenigstens eine der folgenden Substanzen: essbare organische Säure, Tensid, Emulgator, Süssstoff, Aromastoff und Suspensionshilfsmittel - alle oder einzelne gegebenenfalls in Lösung, sowie gegebenenfalls weitere - insbesondere lösliche und/oder grobkörnige - Wirkstoffe zugefügt und untermischt werden, worauf die Mischung abschliessend endgetrocknet wird.
